**Europäisches Patentamt**

**European Patent Office**

**Office européen des brevets**

(19)

(11) Veröffentlichungsnummer: **0 126 315**
**B1**

(12) **EUROPÄISCHE PATENTSCHRIFT**

(45) Veröffentlichungstag der Patentschrift:
14.01.87

(51) Int. Cl.⁴: **A 61 K 31/44**

(21) Anmeldenummer: **84104563.6**

(22) Anmeldetag: **24.04.84**

(54) **Parenterale Formulierung von Nimodipin, ein Verfahren zu ihrer Herstellung sowie ihre Verwendung bei der Bekämpfung von Erkrankungen.**

(30) Priorität: **06.05.83 DE 3316510**

(43) Veröffentlichungstag der Anmeldung:
**28.11.84 Patentblatt 84/48**

(45) Bekanntmachung des Hinweises auf die Patenterteilung:
**14.01.87 Patentblatt 87/3**

(84) Benannte Vertragsstaaten:
**AT BE CH DE FR GB IT LI NL SE**

(56) Entgegenhaltungen:
**EP-A-0 004 650**
**GB-A-1 358 951**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Hoff, Dieter, Dr., Edmund- Husserl-Strasse 19, D-5090 Leverkusen 3 (DE)**

EP 0 126 315 B1

## Beschreibung

Die Erfindung betrifft eine parenterale Formulierung von Nimodipin in einem Lösungsmittel und ein Verfahren zu ihrer Herstellung.

Nimodipin ist ein Dihydropyridinderivat mit der Bezeichnung 1,4-Dihydro-2,6-dimethyl-4-(3-nitrophenyl)-pyridin-3,5-dicarbonsäure-3-β-methoxyethylester-5-isopropylester und aus der DE-OS 2 815 578 und aus EP-A-1 4650 bekannt. Aus den genannten Anmeldungen geht ebenfalls die Verwendung von Nimodipin bei der Behandlung von cerebralen Durchblutungsstörungen hervor.

Es besteht ein Bedürfnis in der Medizin, Parenterale Formulierungen von Nimodipin bereitzustellen, da bewußtlose oder narkotisierte Patienten in diesem Zustand keinen Wirkstoff zu sich nehmen können.

Die Erfindung betrifft somit parenterale Formulierungen enthaltend

a) 0,02 Gewichtsprozent gelöstes Nimodipin bezogen auf 100 Gewichtsteile eines Lösungsmittels bestehend aus

b) 17 - 20 Gewichtsprozent Polyethylenglykol mit mittlerem Molekulargewicht von 200, 400 und/oder 600,

20 Gewichtsprozent Ethanol

ca. 60 Gewichtsprozent Wasser sowie gegebenenfalls geringe Mengen üblicher Hilfs- und/ oder Zuschlagstoffe.

Diese parenteralen Formulierungen werden hergestellt, indem man 0,02 Gewichtsteile Nimodipin in einem Lösungsmittel enthaltend 17 bis 20 Gewichtsteile Polyethylenglykol mit mittlerem Molekulargewicht von 200, 400 und/oder 600 und 20 Gewichtsteile Ethanol löst, gegebenenfalls geringe Mengen üblicher Hilfs- und/oder Zuschlagstoffe zugibt und anschließend mit Wasser auf 100 Gewichtsteile auffüllt.

Unter den Begriff parenteral wird insbesondere intravenös, intraarteriel und intrazisternal verstanden.

Da Nimodipin im hellen Sonnenlicht lichtempfindlich ist, kann es sich als notwendig erweisen, daß der Lösung als Lichtschutz und zwecks Stabilisierung Farbstoffe zugesetzt werden müssen. Geeignete Farbstoffe können beispieleweise Apocarotenal, Canthaxanthin, Tartrazin, Amaranth, Erythrosin, insbesondere jedoch Gelborange S sein. Die Konzentration des jeweiligen Farbstoffes - falls eingesetzt - beträgt 0,01-0,5 Gew.-%, vorzugsweise 0,1 - 0,4 Gew.-%, bezogen auf die Lösung.

Ein Lichtschutz ist ebenfalls möglich, indem man statt der Lösung Injektionsflaschen oder Ampullen lichtundurchlässig ausrüstet, so daß Licht einer bestimmten Wellenlänge nicht oder kaum hindurchscheint.

Zur Stabilisierung der Lösung kann es erforderlich sein, übliche Stabilisatoren zuzugeben, beispielsweise Natriumcitrat tertiär von 0,15 - 2,5, vorzugsweise 1,8 - 2,2 Gew.-% und/oder Citronensäure von 0,2 - 0,6 vorzugsweise 0,2 - 0,5 Gew.-%, bezogen auf 100 Gewichtsteile Lösungsmittel.

Die erfindungsgemäße Formulierung eignet sich zur Behandlung von akuten cerebralen Durchblutungsstörungen wie beispielsweise ischämische neurologische Defizite infolge cerebraler Vasospasmen, Schädel-Hirn-Trauma, ischämischer, cerebraler Insult oder cerebraler Resuscitation.

Im allgemeinen hat es sich als vorteilhaft erwiesen bei

intravenöser Applikation Mengen von etwa 0,5 - 4 mg pro Stunde, bevorzugt 1 - 3 mg pro Stunde,

intraarterieller Applikation Mengen von etwa 0,05 - 0,5 mg pro Stunde, bevorzugt 0,1 - 0,25 mg pro Stunde,

intrazisternaler Applikation Mengen von etwa 100 bis 300 mikrogramm

an Nimodipin zur Erzielung wirksamer Ergebnisse zu verabreichen.

Es kann gegebenenfalls erforderlich sein, von den genannten Mengen abzuweichen, und zwar in Abhängigkeit vom Körpergewicht, aber auch aufgrund des individuellen Verhaltens gegenüber dem Medikament bzw. dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überechritten werden muß.

## Klinische Ergebnisse

Bei prophylaktischer intraoperativ intrazisternaler Gabe von 200 µg Nimodipin und anschließender mehrtägiger intravenöser Dauerinfusion von 1 -3 mg Nimodipin pro Stunde bei Patienten mit einer Subarachnoidalblutung wurden eindeutig neurologische Ausfälle infolge zerebraler Gefäßspasmen verhindert und die Mortalität gesenkt. In einer Serie von 70 Patienten traten keine neurologischen Defizite auf und es verstarb kein Patient. In ähnlich zusammengesetzten historischen Vergleichskollektiven sind dagegen bei 10 - 32 % der Patienten schwere ischämische Komplikationen, die bis zum Tode führen beschrieben.

In einer zweiten Versuchsserie, in der zur Behandlung neurologischer Defizite infolge zerebraler Vasospasmen nach einer Subarachnoidalblutung Nimodipin mehrtägig in Dosen von 0,5 - 3 mg pro Stunde intravenös infundiert wurde, betrug die Mortalität 18 %, während in der Literatur bei diesem Erkrankungebild eine Mortalität von 20 - 50 % angegeben wird. Obwohl es sich um ein negativ selektiertes Patientengut handelte, hatten sich am Behandlungsende zwei Drittel der Patienten vollständig erholt oder deutlich gebessert.

Den Untersuchungen lag Beispiel 1 zugrunde.

**Beispiele:**

| 1. | Nimodipin | 0,200 g |
|---|---|---|
| | Ethanol 96 % | 200,000 g |
| | Polyethylenglykol 400 | 170,000 g |
| | Natriumcitrat tertiär | 2,000 g |
| | Citronensäure | 0,300 g |
| | Wasser für Injektionszwecke | 622,300 g |
| | | 994,800 g |

| 2. | Nimodipin | 0,200 g |
|---|---|---|
| | Ethanol 96 % | 200,000 g |
| | Polyethylenglykol 200 | 200,000 g |
| | Natriumcitrat tertiär | 2,000 g |
| | Citronensäure | 0,300 g |
| | Wasser für Injektionszwecke | 599,440 g |
| | | 1001,940 g |

| 3. | Nimodipin | 0,200 g |
|---|---|---|
| | Ethanol 96 % | 200,000 g |
| | Polyethylenglykol 600 | 170,000 g |
| | Natriumcitrat tertiär | 2,000 g |
| | Citronensäure | 0,300 g |
| | Wasser für Injektionszwecke | 622,300 g |
| | | 994,800 g |

**0 126 315**

**Patentansprüche**

1. Parenterale Formulierung enthaltend
a) 0,02 Gewichtsprozent gelöstes Nimodipin bezogen auf 100 Gewichtsteile eines Lösungsmittels bestehend aus
b) 17 - 20 Gewichtsprozent Polyethylenglykol mit mittlerem Molekulargewicht von 200, 400 und/oder 600,
20 Gewichtsprozent Ethanol
ca. 60 Gewichtsprozent Wasser
sowie gegebenenfalls geringe Mengen üblicher Hilfsund/oder Zuschlagstoffe.

2. Parenterale Formulierung gemäß Anspruch 1, dadurch gekennzeichnet, daß das Lösungsmittel 17 Gewichtsprozent Polyethylenglykol mit mittlerem Molekeulargewicht 400 und 20 Gewichtsprozent Ethanol enthält. [Verfahren zur Herstellung von parenteralen Formulierungen enthaltend 0,02 Gewichtsteile gelöstes Nimodipin bezogen auf 100 Gewichtsteile eines Lösungsmittels bestehend aus 17 - 20 Gewichtsprozent Polyethylenglykol mit mittlerem Molekulargewicht von 200, 400 und/oder 600, 20 Gewichtsprozent Ethanol, ca. 60 Gewichtsprozent Wasser sowie gegebenenfalls geringe Mengen üblicher Hilfs- oder Zuschlagstoffe, dadurch gekennzeichnet, daß man 0,02 Gewichtsteile Nimodipin in einem Lösungsmittel enthaltend 17 - 20 Gewichtsteile Polyethylenglykol mit mittlerem Molekulargewicht von 200, 400 und/oder 600 und 20 Gewichtsteile Ethanol löst, gegebenenfalls geringe Mengen üblicher Hilfs- und/oder Zuschlagstoffe zugibt und anschließend mit Wasser auf 100 Gewichtsteile auffüllt.] geringe Mengen üblicher Hilfs- und/oder Zuschlagstoffe zugibt und anschließend mit Wasser auf 100 Gewichtsteile auffüllt.

4. Verfahren gemäß Anspruch 3, dadurch gekennzeichnet, daß man 0,02 Gewichtsprozent Nimodipin, 17 Gewichtsprozent Polyethylenglykol 400 und 20 Gewichtsprozent Ethanol verwendet.

**Revendications**

1 - Formulation parentérale contenant a) 0,02% en poids de Nimodipine dissoute pour 100 parties en poids d'un solvant consistant en
b) 17 à 20% en poids d'un polyéthylèneglycol ayant un poids mohéculaire moyen de 200, 400 et/ou 600, 20% en poids d'éthanol
environ 60% en poids d'eau,
de même qu'éventuellement de petites quantités d'auxiliaires et/ou additifs usuels.

2 - Formulation parentérale selon la revendication 1, caractérisée en ce que le solvant contient 17% en poids de polyéthylèneglycol ayant un poids moléculaire moyen de 400 et 20% en poids d'éthanol.

3 - Procédé de fabrication de formulations parentérales contenant 0,02 partie en poids de Nimodipine dissoute pour 100 parties d'un solvant consistant en 17-20% en poids de polyéthylèneglycol ayant un poids moléculaire moyen de 200, 400 et/ou 600, 20% en poids d'éthanol, environ 60% en poids d'eau, ainsi éventuellement que de faibles quantités d'auxiliaires ou additifs usuels, caractérisé en ce qu'on dissout 0,02 partie en poids de Nimodipine dans un solvant contenant 17-20 parties en poids d'un polyéthylèneglycol de poids moléculaire moyen 200, 400 et/ou 600 et 20 parties en poids d'éthanol, on ajoute éventuellement de petites quantités d'auxiliaires et/ou additifs usuels et complète ensuite avec de l'eau à 100 parties en poids.

4 - Procédé selon la revendication 3, caractérisé en ce qu'on utilise 0,02% en poids de Nimodipine, 17% en poids de polyéthylèneglycol ayant un poids mocéculaire moyen de 400 et 20% en poids d'éthanol.

**Claims**

1. Parenteral formulation containing
a) 0.02 per cent by weight of dissolved nimodipine based on 100 parts by weight of a soivent consisting of
b) 17 - 20 per cent by weight of polyethylene glycol having an average molecular weight of 200, 400 and/or 500, 20 per cent by weight of ethanol,
about 50 per cent by weight of water
and, optionally, small amounts of customary auxiliaries and/oradditives.

2. Parenteral formulation according to Claim 1, characterised in that the solvent contains 17 per cent by weight of polyethylene glycol having an average molecular weight of 400 and 20 per cent by weight of ethanol.

3. Process for the preparation of parenteral formulations containing 0.02 parts by weight of dissolved nimodipine based on 100 parts by weight of a solvent consisting of 17 - 20 per cent by weight of polyethylene glycol having an average molecular weight of 200, 400 and/or 500, 20 per cent by weight of ethanol, about 50 per cent by weight of water and, optionally, small amounts of customary auxiliaries or additives, characterised in that 0.02 parts by weight of nimodipine are dissolved in a solvent containing 17 - 20 parts by weight of polyethylene glycol having an average molecular weight of 200, 400 and/or 500 and 20 parts by weight of ethanol, small amounts of customary auxiliaries and/or additives are optionally added and water is then added to make up to 100 parts by weight.

4. Process according to Claim 3, characterised in that 0.02 per cent by weight of nimodipine, 17 per cent by weight of polyethylene glycol having an average molecular weight of 400 and 20 per cent by weight of ethanol are used.